(19) Europäisches Patentamt
European Patent Office
Office européen des brevets

(11) Veröffentlichungsnummer : **0 031 109**
**B1**

(12) # EUROPÄISCHE PATENTSCHRIFT

(45) Veröffentlichungstag der Patentschrift : **11.05.83**

(51) Int. Cl.³ : **C 07 C129/12**, A 01 N 47/44

(21) Anmeldenummer : **80107880.9**

(22) Anmeldetag : **13.12.80**

(54) **Neue Guanidiniumverbindungen, Verfahren zu deren Herstellung und deren Verwendung als mikrobiozide Mittel.**

(30) Priorität : **22.12.79 DE 2952167**

(43) Veröffentlichungstag der Anmeldung : **01.07.81 Patentblatt 81/26**

(45) Bekanntmachung des Hinweises auf die Patenterteilung : **11.05.83 Patentblatt 83/19**

(84) Benannte Vertragsstaaten : **BE CH DE FR GB IT LI NL SE**

(56) Entgegenhaltungen : **GB A 1 294 443**

(73) Patentinhaber : **HOECHST AKTIENGESELLSCHAFT**
**Postfach 80 03 20**
**D-6230 Frankfurt/Main 80 (DE)**

(72) Erfinder : **Diery, Helmut, Dr.**
**Theresenstrasse 45**
**D-6233 Kelkheim (Taunus) (DE)**
Erfinder : **Wagemann, Wolfgang, Dr.**
**Beektwiete 2a**
**D-2071 Tremsbüttel (DE)**
Erfinder : **Bücking, Hans-Walter, Dr.**
**In den Padenwiesen 30**
**D-6233 Kelkheim (Taunus) (DE)**
Erfinder : **Hille, Martin, Dr.**
**In den Eichen 46**
**D-6237 Liederbach (DE)**
Erfinder : **Wallhäusser, Karl Heinz, Prof., Dr.**
**Lessingstrasse 20**
**D-6238 Hofheim am Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 031 109 B1

Neue Guanidiniumverbindungen, Verfahren zu deren Herstellung und deren Verwendung als mikrobiozide Mittel

Gegenstand der Erfindung sind Guanidiniumsalze der Formel (1)

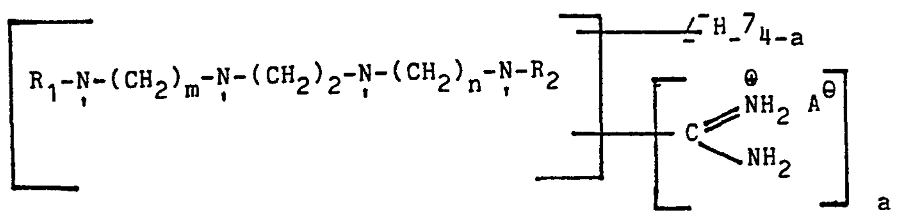

(1)

worin $R_1$ und $R_2$ gleich oder verschieden sind und Alkyl, 2-Hydroxyalkyl oder Alkenyl mit jeweils 8-18 C-Atomen oder $C_8$-$C_{18}$-Alkoxipropyl, m und n 2 oder 3, a eine Zahl von 1 bis 4 und A ein Anion bedeutet.

Bevorzugt sind solche Verbindungen der Formel 1, worin $R_1$, $R_2$ und a die obige Bedeutung haben und m und n 3 und A das Acetation bedeuten.

Diese Verbindungen werden hergestellt, indem man 1 Mol einer Verbindung der Formel (2)

$$R_1—NH—(CH_2)_m—NH—(CH_2)_2—NH—(CH_2)_n—NH—R_2 \qquad (2)$$

in Gegenwart einer Säure mit 1 bis 4 Mol Cyanamid umsetzt. Die Reaktion wird vorzugsweise in Wasser oder niederen Alkoholen bei Temperaturen von 40 bis 100 °C, vorzugsweise 50-90 °C durchgeführt. Dabei geht man zweckmäßigerweise so vor, daß man zunächst die Säure zu dem Amin der Formel 2 gibt und so das entsprechende Salz erzeugt. Anschließend gibt man zu diesem Salz eine wässrige oder alkoholische Lösung von Cyanamid und läßt ca. 2 bis 5 Stunden unter Rühren reagieren. Als Säuren dienen Mineralsäure, Essigsäure, Milchsäure, Ameisensäure, Gluconsäure oder andere organische Säuren. Vorzugsweise wird Essigsäure verwendet.

Eine andere Möglichkeit zur Herstellung der Verbindungen der Formel 1 besteht darin, daß man eine Verbindung der Formel 2 mit Methoxyharnstoff-sulfat oder S-Methylthioharnstoff-sulfat in wässriger und/oder alkoholischer Lösung umsetzt. In diesem Fall entfällt die gesonderte Zugabe einer Säure wie es bei der Umsetzung mit Cyanamid der Fall ist.

Entsprechend dem Molverhältnis von Amin der Formel 2 und Cyanamid, Methoxyharnstoff-sulfat bzw. S-Methylthioharnstoffsulfat lassen sich bis zu vier Guanidinium-Gruppen einführen. Da alle vier Aminogruppen gleichartig sind, läßt sich bei Verwendung von weniger als vier Mol Cyanamid, Methoxyharnstoff-sulfat bzw. S-Methylthioharnstoff-sulfat die genaue Position der Guanidinium-Gruppen nicht feststellen.

Die als Ausgangsprodukt dienenden Amine der Formel 2 werden nach dem in der deutschen Patentanmeldung P 29 38 710.8 beschriebenen Verfahren erhalten, indem man Alkylalkylendiamine der Formel

$$R_1—NH—(CH_2)_m—NH_2, \quad \text{bzw.} \quad R_2—NH—(CH_2)_n—NH_2$$

mit Glyoxal zu Bisaldiminen der Formel

$$R_1—NH—(CH_2)_m—N = CH—CH = N—(CH_2)_n—NH—R_2$$

umsetzt und dieses Bisaldimin dann katalytisch reduziert.

Die erfindungsgemäßen Verbindungen der Formel 1 fallen bei der Synthese als braune, mehr oder weniger viskose Flüssigkeiten an, die in jedem Verhältnis mit Wasser und/oder niederen Alkoholen mischbar sind. Die reinen Produkte können durch Abdestillation der Lösungsmittel in pastöser Form gewonnen werden.

Die Verbindungen zeigen eine ausgeprägte biozide Wirksamkeit gegen grampositive und gramnegative Bakterien sowie gegen Schimmelpilze und Algen. Besonders hervorzuheben ist die hervorragende Wirkung gegen sulfatreduzierende Bakterien vom Typ Desulfovibrio desulfuricans, deren Stoffwechselprodukte, insbesondere Schwefelwasserstoff, für Korrosionsschäden größten Ausmaßes an Erdölfördersonden, Ölfeldleitungen, Rohölaufbereitungsanlagen, Flutwasserleitungen und Flutwassereinpreßsonden verantwortlich sind. Zudem entwickeln Bakterien dieses Typs sehr schnelle Resistenz, so daß ein häufiger Wechsel der eingesetzten Bakterizide vonnöten ist, was ein breites Angebot an derartigen Produkten verlangt. Die hervorragende Löslichkeit der erfindungsgemäßen Verbindungen erlaubt es, hochprozentige Konzentrate z. B. in Alkoholen einzusetzen, die an Ort und Stelle mit Wasser beliebig verdünnt und präzise dosiert werden können, was für den Einsatz im Erdölsektor von großem Vorteil ist.

Infolge ihres ausgezeichneten großen Wirkungsspektrums, ihrer guten Wasserlöslichkeit und Hautverträglichkeit können die erfindungsgemäßen Verbindungen außerdem vorteilhaft ganz allgemein als Desinfektionsmittel verwendet werden. Die gute Kurzzeitwirkung erlaubt den Einsatz als Hände- und Flächendesinfektionsmittel.

Flüssige Zubereitungen solcher Desinfektionsmittel werden im allgemeinen in Wasser oder niederen Alkoholen wie z. B. Isopropanol oder Wasser/Alkohol-Gemischen hergestellt. Sie enthalten 5 bis 70 Gew.-%, vorzugsweise 20 bis 55 Gew.-% der erfindungsgemäßen Guanidiniumverbindungen. Für die Herstellung solcher flüssiger Zubereitungen kann man direkt von den Lösungen ausgehen, die bei der Synthese dieser Guanidiniumverbindungen anfallen. Man verdünnt diese Lösungen durch Zugabe von Wasser bzw. Alkohol auf die gewünschte Endkonzentration. Selbstverständlich können in solchen Formulierungen außerdem noch weitere Substanzen und Hilfsmittel enthalten sein, wie sie üblicherweise in solchen Zubereitungen mitverwendet werden. Hierzu gehören z. B. kationische oder nichtionische oberflächenaktive Substanzen, Elektrolyte, Komplexbildner, Lösungsvermittler, niedere Aldehyde wie z. B. Formaldehyd, Glyoxal oder Glutardialdehyd sowie Farb- und Duftstoffe. Diese Zusätze dienen beispielsweise zur Verbesserung des Netzvermögens, der Härtestabilität, zur Viskositätseinstellung und zur Erhöhung der Kältestabilität der Lösungen.

Herstellungsbeispiel 1

252 g (0,4 Mol) N,N'-Dilaurylaminopropylethylendiamin werden auf 75 °C erwärmt und bei dieser Temperatur 420 g (1,4 Mol) 20 %ige Essigsäure zugetropft. Zur Vervollständigung der Aminsalzbildung wird 15 Minuten bei 75 °C nachgerührt. Dann tropft man bei der gleichen Temperatur 116 g (1,38 Mol) 50 %ige wäßrige Cyanamid-Lösung hinzu und rührt 4 Stunden nach. Die resultierende klare rötlichbraune Lösung hat eine pH-Wert von 7. Mit 50 %iger Essigsäure wird auf 6,5 eingestellt. Es ist kein freies Cyanamid mehr nachzuweisen. Das Produkt ist klarflüssig und 45 %ig.

Herstellungsbeispiel 2

54,2 g (0,085 Mol) N,N'-Dilaurylaminopropylethylendiamin werden in 320 g 6 %ige Essigsäure gegeben und unter Erwärmen bei gleichzeitiger Aminsalzbildung gelöst. Bei 70 °C werden innerhalb von 20 Minuten 12,6 g (0,3 Mol) Cyanamid, das vorher in 15 ml Wasser gelöst wurde, zugegeben. Man rührt 5 Stunden bei 70 °C nach. Man erhält eine klare dünnflüssige 23 %ige Lösung mit einem pH-Wert von 6,4 (1 %ig gemessen). Es ist kein freies Cyanamid mehr nachzuweisen.

Herstellungsbeispiel 3

252 g (0,4 Mol) N,N'-Dilaurylaminopropylethylendiamin werden in 80 g Isobutanol auf 70 °C erwärmt. Bei dieser Temperatur werden im Laufe von 20 Minuten 48 g (0,8 Mol) Eisessig zugetropft. Nach kurzem Nachrühren werden 282 g (0,8 Mol) 12 %ige wäßrige Cyanamid-Lösung bei 70-80 °C zugetropft (40 Minuten). Man rührt unter Stickstoff 2 Stunden bei 70-80 °C, anschließend 2 Stunden bei 90-95 °C nach, läßt auf 30-40 °C abkühlen und gibt nacheinander 48 g Eisessig und 60 g Isopropanol zu. Das Produkt ist klarflüssig bei einem Gehalt von 50 %. Freies Cyanamid ist nicht mehr nachzuweisen.

Herstellungsbeispiel 4

57,2 g (0,1 Mol) N,N'-Dilaurylaminopropylethylenediamin werden unter Erwärmen in 20 g Isobutanol gelöst. Innerhalb von 25 Minuten werden bei 70 °C 24 g (0,4 Mol) Essigsäure zugetropft. Man heizt auf 75-80 °C und tropft bei dieser Temperatur 33,6 g (0,4 Mol) 50 %ige wäßrige Cyanamid-Lösung innerhalb von 20 Minuten zu. Man gibt noch 45 g Wasser hinzu und rührt 3 Stunden bei 90-95 °C nach. Nach dem Abkühlen auf 35 °C werden 15 g Isopropanol zugegeben. Man erhält eine klare flüssige 53 %ige Lösung vom pH-Wert 6,4.

Die Konstitution der nach den Beispielen 1 bis 4 erhaltenen Verbindungen der Formel 1 ist aus der folgenden Tabelle ersichtlich :

Beispiel 1 bis 4

$R_1 = R_2 =$ Laurylalkyl (75 % $C_{12}$ ; 25 % $C_{14}$)
$m = n = 3$
A = Acetat

Beispiel 1 : a = 3,5
Beispiel 2 : a = 3,5
Beispiel 3 : a = 2
Beispiel 4 : a = 4

## Anwendungsbeispiel 1

Bakterizide und fungizide Wirkung : Suspensionsversuch mit ca. $10^6$ Keimen/ml, Einwirkzeit 24 und 48 Stdn. bei 20 °C, Angabe der minimalen mikrobiziden Konzentration in µg/ml.

| Produkt aus Beispiel | Staph. aur. | E.coli | Ps. aerug. | Prot. vulg. | Cand. alb. |
|---|---|---|---|---|---|
| 1: 24 Stdn. | 31 | 31 | 16 | 16 | 31 |
| 2: 24 Stdn. | 31 | 31 | 16 | 62 | 16 |
| 3: 24 Stdn. | 16 | <4 | 8 | <4 | 16 |
| 4: 24 Stdn. | 125 | 62 | 31 | 8 | 16 |
| 1: 48 Stdn. | 8 | <4 | 16 | 8 | 8 |
| 2: 48 Stdn. | <4 | <4 | 16 | 16 | 16 |
| 3: 48 Stdn. | <4 | <4 | <4 | <4 | <4 |
| 4: 48 Stdn. | 8 | <4 | 16 | <4 | 8 |
| Vergleichsprodukt: 24 Stdn. | 8 | 16 | 16 | 125 | 125 |
| Vergleichsprodukt: 48 Stdn. | <4 | 8 | 16 | 16 | 16 |

Vergleichsprodukt : Cocosalkyl-dimethyl-benzyl-ammoniumchlorid

## Anwendungsbeispiel 2

Bakterizide und fungizide Kurzzeitwirkung-Suspensionsversuch, Bedingungen wie Beispiel 1, Einwirkzeit 1 Stunde und 4 Stunden. Angabe der minimalen Wirkkonzentration in µg/ml

| Produkt aus Beispiel | Staph. aur. | E.coli | Ps. aerug. | Prot. vulg. | Cand. alb. |
|---|---|---|---|---|---|
| 2: 1 Std. | 62 | 500 | 62 | 2000 | 500 |
| 4: 1 Std. | 1000 | 1000 | 125 | 1000 | 500 |
| 2: 4 Std. | 62 | 16 | 62 | 31 | 500 |
| 4: 4 Std. | 1000 | 125 | 125 | 62 | 62 |
| Vergleichsprodukt: 1 Std. | 250 | 16 | 32 | 16 | 125 |
| Vergleichsprodukt: 4 Stdn. | 16 | 16 | 16 | 16 | 62 |

Vergleichsprodukt : Cocosalkyldiamin-biguanidiniumacetat

## Anwendungsbeispiel 3

Bakterizide Wirkung gegen Desulfovibrio desulfuricans-Suspensionsversuch, Bedingungen wie Beispiel 1. Angabe der minimalen mikrobioziden Konzentration in µg/ml

| Produkt aus Beispiel | Desulf. des. Nr. 39 | Desulf. des. Nr. 85 |
|---|---|---|
| 1: 24 Stdn. | 8 | 8 |
| 1: 48 Stdn. | 8 | 8 |
| 2: 24 Stdn. | 62 | 62 |

(Fortsetzung)

| Produkt aus Beispiel | Desulf. des. Nr. 39 | Desulf. des. Nr. 85 |
|---|---|---|
| 2: 48 Stdn. | 16 | 8 |
| 3: 24 Stdn. | 8 | < 4 |
| 3: 48 Stdn. | 8 | < 4 |
| 4: 24 Stdn. | < 4 | < 4 |
| 4: 48 Stdn. | < 4 | < 4 |
| Vergl.: 24 Stdn. | 64 | 64 |
| Vergl.: 48 Stdn. | 64 | 32 |

Vergleichsprodukt : wie Anwendungsbeispiel 1

Anwendungsbeispiel 4

Prüfung der Flächendesinfektionswirkung an Staph.aur. und Klebs.pneum., Beimpfung von 50 × 50 mm großen Stücken aus Schichtpreßstoff, PVC-Boden oder OP-Fliesen. Keimgehalt pro ml CSL-Suspension : $10^8$-$10^9$. Nach Antrocknung der Keimsuspension (90 Min.) werden die Flächen aus einer Entfernung von 30-40 cm mit dem Mittel besprüht.

Zusammensetzung der Formulierungen :

A : 11,1 % Produkt aus Herst. Beisp. 1
5 % Nonylphenol 10 EO-polyglycolether
ad 100 % Wasser

B : 11,1 % Produkt aus Herst. Beisp. 1
5 % Nonylphenol 10 EO-polyglycolether
5 % Formalin 35 %ig
4 % Glyoxal 40 %ig
5 % Isopropanol
ad 100 % Wasser

Prüfergebnis

| Formulierung verdünnt mit $H_2O$ | Restkeimgehalt* (KBE) Staph.aur. 1 | 2 | 4 | 6 Stdn. | Klebs.pneum. 1 | 2 | 4 | 6 Stdn. |
|---|---|---|---|---|---|---|---|---|
| A 1:10 | 0 | 0 | 0 | 0 | 100 | 50 | 50 | 50 |
| B 1:10 | 0 | 0 | 0 | 0 | 0 | 0 | 0 | 0 |
| A 1:100 | 30 | 0 | 0 | 0 | 30 | 20 | 20 | 20 |
| B 1:100 | 20 | 20 | 20 | 20 | 100 | 100 | 100 | 100 |

* entsprechend den Empfehlungen für die Prüfung und Bewertung der Wirksamkeit chemischer Desinfektionsverfahren (Zbl. Bakt. Hyg. I Abt. Orig. B 165, 335-380 (1977)

**Ansprüche**

1. Guanidiniumverbindungen der Formel (1)

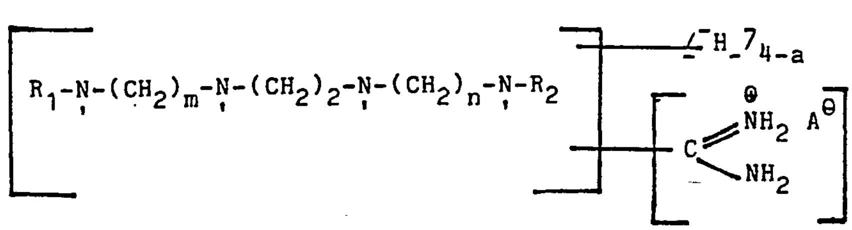

(1)

worin $R_1$ und $R_2$ gleich oder verschieden sind und Alkyl, 2-Hydroxyalkyl oder Alkenyl mit jeweils 8-18 C-Atomen oder $C_8$-$C_{18}$-Alkoxipropyl, m und n 2 oder 3, a eine Zahl von 1 bis 4 und A ein Anion bedeutet.

2. Verfahren zur Herstellung der Guanidiniumverbindungen nach Anspruch 1, dadurch gekennzeichnet, daß man 1 Mol einer Verbindung der Formel 2

$$R_1—NH—(CH_2)_m—NH—(CH_2)_2—NH—(CH_2)_n—NH—R_2 \qquad (2)$$

mit 1 bis 4 Mol Methoxyharnstoff-sulfat oder S-Methylthioharnstoff-sulfat oder in Gegenwart einer Säure mit 1 bis 4 Mol Cyanamid umsetzt.

3. Verwendung der Guanidiniumverbindungen nach Anspruch 1 als mikrobiozide Mittel.

**Claims**

1. Guanidinium compounds of the formula (1)

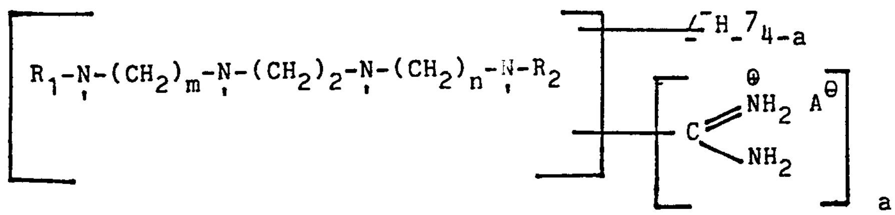

(1)

in which $R_1$ and $R_2$ are identical or different and denote alkyl, 2-hydroxyalkyl or alkenyl each having from 8 to 18 carbon atoms, or $C_8$-$C_{18}$-alkoxylpropyl, m and n are 2 or 3, a is a number from 1 to 4 and A denotes an anion.

2. Process for the manufacture of guanidinium compounds as defined in claim 1, which comprises reacting 1 mol of a compound of the formula (2)

$$R_1—NH—(CH_2)_m—NH—(CH_2)_2—NH—(CH_2)_n—NH—R_2 \qquad (2)$$

with 1 to 4 mols of methoxyurea sulfate or S-methylthiourea sulfate or, in the presence of an acid, with 1 to 4 mols of cyanamide.

3. Use of the guanidinium compounds as defined in claim 1 as microbiocidal agents.

**Revendications**

1. Composés de guanidiniums répondant à la formule (1)

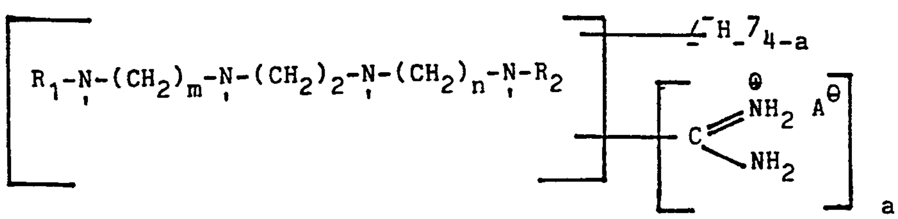

(1)

dans laquelle $R_1$ et $R_2$ sont identiques ou différents l'un de l'autre et représentent chacun un radical alkyle, un radical hydroxy-2 alkyle ou un radical alcényle contenant chacun de 8 à 18 atomes de carbone ou un radical alcoxypropyle contenant de 8 à 18 atomes de carbone dans sa partie alcoxy, m et n sont égaux à 2 ou à 3, a représente un nombre de 1 à 4 et A représente un anion.

2. Procédé de préparation des composés de guanidiniums selon la revendication 1, procédé caractérisé en ce qu'on fait réagir 1 mole d'un composé de formule (2)

$$R_1—NH—(CH_2)_m—NH—(CH_2)_2—NH—(CH_2)_n—NH—R_2 \qquad (2)$$

avec de 1 à 4 moles de sulfate de méthoxy-urée ou de sulfate de S-méthyl-thio-urée, ou, en présence d'un acide, avec de 1 à 4 moles de cyanamide.

3. Application des composés de guanidiniums selon la revendication 1 comme agents microbicides.